(19) Europäisches Patentamt
European Patent Office
Office européen des brevets

(11) **EP 3 919 083 B1**

(12) **EUROPÄISCHE PATENTSCHRIFT**

(45) Veröffentlichungstag und Bekanntmachung des Hinweises auf die Patenterteilung:
**11.10.2023   Patentblatt 2023/41**

(21) Anmeldenummer: **21175986.5**

(22) Anmeldetag: **26.05.2021**

(51) Internationale Patentklassifikation (IPC):
**A61L 2/00** *(2006.01)*       **A61L 2/08** *(2006.01)*

(52) Gemeinsame Patentklassifikation (CPC):
**A61L 2/0023; A61L 2/085**

(54) **VORRICHTUNG UND VERFAHREN ZUM INAKTIVIEREN VON MIKROORGANISMEN**

DEVICE AND METHOD FOR INACTIVATING MICROORGANISMS

DISPOSITIF ET PROCÉDÉ D'INACTIVATION DES MICRO-ORGANISMES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **26.05.2020   AT 504562020**

(43) Veröffentlichungstag der Anmeldung:
**08.12.2021   Patentblatt 2021/49**

(73) Patentinhaber: **Pürker, Robert**
**8643 Kindberg (AT)**

(72) Erfinder: **Pürker, Robert**
**8643 Kindberg (AT)**

(74) Vertreter: **Wirnsberger & Lerchbaum**
**Patentanwälte OG**
**Mühlgasse 3**
**8700 Leoben (AT)**

(56) Entgegenhaltungen:
**WO-A1-00/38742       CN-A- 106 213 836**
**CN-A- 110 839 814**

**Beschreibung**

Die Erfindung betrifft eine Vorrichtung zum Inaktivieren von Mikroorganismen die sich auf und/oder in einem Objekt befinden, insbesondere von Bakterien und/oder Viren, aufweisend eine Grundfläche, eine Deckfläche und zumindest zwei Seitenflächen, welche die Grundfläche und die Deckfläche miteinander verbinden, wodurch ein Volumen zur Aufnahme des Objektes gebildet ist.

Weiter betrifft die Erfindung eine Verwendung einer derartigen Vorrichtung.

Zudem betrifft die Erfindung ein Verfahren zum Inaktivieren von Mikroorganismen, insbesondere von Bakterien und/oder Viren, welche eine Deckfläche, zumindest zwei Seitenflächen und ein dadurch gebildetes Volumen zur Aufnahme eines Objektes umfasst, wobei auf und/oder in den Objekten befindliche Mikroorganismen bei erhöhten Temperaturen inaktiviert werden.

Aus dem Stand der Technik sind verschiedene Vorrichtungen zum Inaktivieren von Mikroorganismen, insbesondere von Krankheitserregern, bekannt geworden. Um eine entsprechende Inaktivierung sicherzustellen, werden hierbei häufig Chemikalien, Strahlung oder Hitze eingesetzt. Dabei sieht eine Inaktivierung mithilfe von Chemikalien oft den Einsatz fein verteilter Partikel, beispielsweise Aerosole, oder gasförmiger Stoffe vor, birgt jedoch den Nachteil, lediglich oberflächlich eine inaktivierende Wirkung erzielen zu können. Abhängig von der Chemikalie und der Oberfläche kann auch eine Reaktion stattfinden, durch welche die Oberfläche gegebenenfalls sogar beschädigt wird. Entsprechend ist bei derartigen Vorrichtungen auf Wechselwirkungen zwischen der Chemikalie und dem Material der Oberfläche zu achten.

Darüber hinaus lässt sich eine oberflächliche Inaktivierung von Mikroorganismen auch mit Strahlung im ultravioletten Wellenlängenbereich erzielen, da diese für gewöhnlich eine geringe Eindringtiefe aufweist. Strahlung mit einer entsprechenden Eindringtiefe, wie zum Beispiel ionisierende Strahlung, findet aufgrund ihres Gefahrenpotenzials nur in wenigen Fällen Anwendung. Aus der CN106213836A und WO00/38742A1 sind Vorrichtungen, Verwendungen solcher Vorrichtungen und Verfahren zum Inaktivieren von Mikroorganismen die sich auf und/oder in einem Objekt befinden, bekannt, welche mittels Infrarotstrahlvorrichtungen funktionieren.

Der Einsatz von Hitze ermöglicht bei geringem Gefahrenpotenzial eine effiziente Dekontamination einer Vielzahl von Objekten, insbesondere von verpackten oder mit Hohlräumen versehenen Objekten, sofern diese eine entsprechende Temperaturbeständigkeit aufweisen. Deshalb gehören dem Stand der Technik auch Vorrichtungen an, welche eine Inaktivierung von Mikroorganismen mittels Hitze durchführen. Zur Wärmeerzeugung sehen entsprechende Dokumente neben Heizspiralen und Mikrowellengeneratoren auch Infrarotstrahler vor. Letztere werden hierbei oft ausschließlich zur Erwärmung der zu dekontaminierenden Oberflächen genutzt, sodass ein zeit- und energiesparender Inaktivierungsvorgang sichergestellt werden kann. Hierbei sind aus dem Stand der Technik auch mobile Vorrichtungen zum Inaktivieren von Mikroorganismen bekannt, welche den Einsatz von Infrarotstrahlern zum Erhitzen von Objekten vorsehen und damit ausschließlich auf eine zeit- und energiesparende Dekontamination der Oberfläche abzielen. Um Mikroorganismen nicht nur oberflächlich, sondern auch im Inneren der Objekte, wie verpackten Produkten, erfolgreich inaktivieren zu können, bedarf es jedoch einer gleichmäßigen Erwärmung der Objekte.

Folglich ist die Aufgabe der Erfindung, eine Vorrichtung der eingangs genannten Art anzugeben, mit welcher eine Inaktivierung von Mikroorganismen an der Oberfläche und gegebenenfalls auch im Inneren eines Objektes ermöglicht wird.

Darüber hinaus ist es Aufgabe der Erfindung, eine Verwendung einer derartigen Vorrichtung anzugeben.

Weiter ist es Aufgabe der Erfindung, ein Verfahren der eingangs genannten Art anzugeben, das unabhängig von der Beschaffenheit eines Objektes eine effiziente Inaktivierung von darauf und/oder darin befindlichen Mikroorganismen ermöglicht.

Die erste Aufgabe wird erfindungsgemäß mit einer Vorrichtung gemäß Anspruch 1 gelöst.

Ein mit der Erfindung erzielter Vorteil ist insbesondere darin zu sehen, dass eine große Bandbreite von Objekten gleichmäßig erwärmt und Mikroorganismen dabei inaktiviert werden können. Zu diesem Zweck wird von zumindest zwei Seitenflächen, einer Deckfläche und einer Grundfläche ein Volumen gebildet. Hierbei können die Seitenflächen einzeln mit Deckfläche und Grundfläche verbunden werden oder alternativ bereits im Vorhinein mit der Deckfläche zu einer Art Haube zusammengefügt werden, welche mit der Grundfläche verbunden wird. Das gebildete Volumen dient nun der Aufnahme von Objekten, wobei deren Anzahl keine Auswirkung auf eine Effizienz der Vorrichtung hat. Folglich spielt es auch keine Rolle, ob ein Objekt in einem anderen Objekt verbaut, einzeln, verpackt oder unverpackt behandelt wird. Eine Hitzebeständigkeit der Objekte ist nur bis zur Betriebstemperatur der Vorrichtung, bevorzugt zwischen 60 °C und 120 °C, besonders bevorzugt zwischen 70 °C und 90 °C, erforderlich und bei den meisten technischen Bauteilen sowie Verpackungsmaterialien ohnehin gegeben. Zum Erwärmen des Volumens wird zumindest eine Inaktivierungseinrichtung eingesetzt, welche bevorzugt vor den Seitenflächen der Vorrichtung angeordnet ist. Hierdurch findet ein Wärmeeintrag in das Volumen statt, sodass die im Volumen befindlichen Objekte gleichmäßig erwärmt werden können. Um eine vollständige Inaktivierung von Mikroorganismen sicherzustellen, beträgt die Verweilzeit der Objekte in einer erfindungsgemäßen Vorrichtung üblicherweise zwischen 30 und 180 Minuten, bevorzugt zwischen 30 und 120 Minuten, besonders bevorzugt zwischen 40 und 80 Minuten. Zudem kann der Wärmeeintrag in das Volumen durch Anordnung von mehreren Inaktivierungseinrichtungen erhöht werden.

Erfindungsgemäß umfasst die Inaktivierungseinrichtung zumindest ein im Volumen vor den Seitenflächen angeordnetes Heizelement zum Beheizen des Volumens und/oder ein Führungsmittel zum Einbringen eines Fluids, insbesondere von Wasser, in das Volumen. Hierdurch können auf und/oder in dem Objekt befindliche Mikroorganismen auf besonders effiziente Weise inaktiviert werden. Entsprechend wird das zumindest eine Heizelement im Volumen vor der Seitenfläche angeordnet, sodass dieses Heizelement das Volumen sowie das im Volumen befindliche Objekt, insbesondere unmittelbar, beheizen kann. Dabei ist das Heizelement vorzugsweise platzsparend und/oder flach ausgebildet, um innerhalb des Volumens besonders viel Platz zum Einbringen von Objekten nutzen zu können. Für gewöhnlich wird das Heizelement vor den Seitenflächen und nahe der Grundfläche der Vorrichtung angeordnet, sodass vom Heizelement abgegebene Wärme innerhalb der Vorrichtung aufsteigen kann. Durch eine derartige Anordnung des Heizelementes kann die abgegebene Wärme von einem im Volumen befindlichen Medium, beispielsweise Umgebungsluft, aufgenommen und mittels Konvektion innerhalb des Volumens verteilt werden. Dabei kann auch das Fluid, welches über das Führungsmittel in das Volumen eingebracht wird, einen Teil des Mediums darstellen und/oder sich mit dem Medium vermischen.

Mit Vorteil wird das Fluid über das Führungsmittel in flüssiger Form eingebracht, wobei dieses zerstäubt und ein Aerosol gebildet werden kann. Entsprechend ist das Führungsmittel vorzugsweise als Leitung, insbesondere als Rohrleitung, mit endseitig angebrachter Düse ausgebildet. Um das Fluid gleichmäßiger in das Volumen einzubringen, können auch mehrere Führungsmittel vorgesehen sein, welche vorzugsweise ortsverschieden, insbesondere nahe unterschiedlicher Flächen der Vorrichtung, im Volumen positioniert sind. Es hat sich bewährt, dass das Führungsmittel als Sprühnebelanlage ausgebildet ist. Diese Sprühnebelanlage weist in der Regel mehrere Leitungen mit Öffnungen auf, an welchen Düsen angeordnet sein können. Alternativ kann das Fluid auch in der Gasphase über das Führungsmittel in das Volumen eingeleitet werden und sich in diesem verteilen. Bevorzugt wird Wasser, insbesondere demineralisiertes Wasser, destilliertes Wasser und/oder Reinstwasser, als Fluid verwendet.

Ist im Volumen kein Heizelement vorgesehen, ist es von Vorteil, das Fluid über mehrere Führungsmittel ortsunterschiedlich in das Volumen einzubringen. Vorzugsweise befindet sich das Fluid hierbei in der Gasphase, sodass dieses sich besonders schnell und effizient im Volumen verteilen kann. Zudem wird bei einer derartigen Ausführung der Vorrichtung bevorzugt ein Fluid verwendet, welches selbst zum Inaktivieren von Mikroorganismen geeignet ist. In solchen Fluiden sind oftmals Bestandteile wie Alkohole oder Aldehyde enthalten.

Es hat sich bewährt, dass das Heizelement zum Erhitzen des Fluids ausgebildet ist. Hierdurch kann eine Wärmeleitfähigkeit des Fluids zur effizienteren Übertragung von Wärme auf das Objekt genutzt und das Inaktivieren von Mikroorganismen auf der Oberfläche des Objektes im Vergleich zu trockenen Bedingungen verbessert werden. Dabei kann das Volumen zunächst über das Heizelement vorgewärmt werden, ehe das Fluid in das Volumen eingebracht wird. Üblicherweise erfolgt das Einbringen des Fluids, insbesondere des Wassers, über mehrere Führungsmittel, um besonders schnell eine gleichmäßige Verteilung des Fluids erzielen zu können. In der Folge wird das im Volumen befindliche Medium samt Fluid vom Heizelement auf Temperaturen zwischen 60 °C und 120 °C, bevorzugt zwischen 70 °C und 90 °C, erhitzt. Dementsprechend werden im Volumen befindliche Objekte vom Heizelement, vom Medium und/oder vom Fluid, vorzugsweise über einen Zeitraum von mindestens 30 Minuten, auf die entsprechende Temperatur erhitzt.

Da im Volumen als Medium zumeist Umgebungsluft vorliegt und das Fluid, bevorzugt Wasser, in dieses eingebracht wird, entsteht im Volumen eine feuchte Atmosphäre. Wird diese Atmosphäre auf die oben angegebene oder höhere Temperaturen erhitzt, spricht man von feuchter Hitze. Dabei kann ein Teil des Fluids, insbesondere bei Verwendung von Wasser, als Flüssigkeit und ein anderer Teil des Fluids als Gas vorliegen. Beim Einbringen von Wasser in das Volumen ergibt sich demnach der Vorteil, dass die im Vergleich zu Umgebungsluft hohe Wärmeleitfähigkeit des Wassers genutzt werden kann. Hierdurch kann die Wärme des Heizelementes besonders effizient auf das Objekt übertragen und auf der Oberfläche des Objektes befindliche Mikroorganismen inaktiviert werden. Durch diese effiziente Übertragung der Wärme auf das Objekt, können auch im Inneren des Objektes befindliche Mikroorganismen auf effiziente Weise inaktiviert werden.

Es hat sich bewährt, das Führungsmittel als Sprühnebelanlage mit einer oder mehreren Öffnungen auszubilden. Vorzugsweise wird das Wasser dabei an unterschiedliche Stellen im Volumen befördert und durch die Öffnungen in das Volumen eingebracht. Für gewöhnlich besteht die Sprühnebelanlage aus zumindest einer Leitung, in welcher das Wasser geführt wird, mit mehreren Öffnungen, an welchen Düsen zum Einsprühen des Wassers in das Volumen angeordnet sind. Um das Einsprühen des Wassers leichter regulieren zu können, kann die Sprühnebelanlage auch eine Pumpe umfassen. Beim Einsprühen des Wassers in das Volumen entsteht ein Aerosol, wodurch eine Wärmeübertragung vom Heizelement auf das Wasser besonders schnell und einfach erfolgen kann. Bevorzugt wird das Aerosol durch die vom Heizelement ausgesandte Wärme erhitzt, sodass im Aerosol enthaltenes Wasser in die Gasphase übergeht. Um das Wasser zumindest teilweise, insbesondere vollständig, in der Gasphase halten zu können, wird die über das Führungsmittel zugeführte Wassermenge derart dosiert, dass eine Übersättigung der im Volumen befindlichen Luft vermieden wird. Dementsprechend wird die eingebrachte Wassermenge an die Temperatur des Volumens angepasst, wodurch ein Überschreiten eines Taupunkts des Wassers und damit eine Kondensation des Wassers an der Oberfläche des Objektes und/oder den Flächen, insbesondere an Innenseiten der Grund-, Deck- und Seitenflächen, der Vorrichtung verhindert werden kann. Um etwaige Rückstände des Wassers am Objekt oder im Volumen reduzieren zu können, kommt vorzugsweise demineralisiertes Wasser, insbesondere destilliertes Wasser oder Reinstwasser, zum Einsatz. Darüber hinaus kann das Wasser auch vor dem Einbringen in das Volumen vom Heizelement erhitzt werden, um das Wasser, insbesondere direkt, als Wasserdampf in das Volumen einzubringen. Entsprechend kann mit der Einbringung des erhitzten Wasserdampfes auch Wärme in das Volumen eingebracht werden.

Erfindungsgemäß ist das Heizelement als Elektroheizer, insbesondere als Heizlüfter ausgebildet. Dadurch kann Wärme besonders effizient in das Volumen eingebracht werden. Hierbei werden in der Regel mehrere Heizelemente in Form von Elektroheizern im Volumen vor einer Seitenwand der Vorrichtung angeordnet. Diese sind vorzugsweise nebeneinander und nahe der Grundfläche positioniert, sodass diese Wärme in einen unteren Bereich des Volumens einbringen können. Üblicherweise sind die Elektroheizer als Heizlüfter ausgebildet, welche ein Heizelement, insbesondere einen Blankdraht oder eine Heizwendel, sowie einen Ventilator aufweisen. Entsprechend können die Elektroheizer auch als Umluftheizelemente oder Heißluftheizelemente bezeichnet werden. Durch den Ventilator wird die Konvektion des Mediums und/oder des Fluids gefördert, sodass die Temperatur im Volumen besonders schnell verändert werden kann. Zudem kann die Temperatur im Volumen durch eine derartige Anordnung der Elektroheizer besonders konstant gehalten werden. Hierbei kommen Elektroheizer mit einer Leistung von 1 Kilowatt (kW) bis 15 kW, bevorzugt 1 kW bis 10 kW, besonders bevorzugt 2 kW bis 5 kW, zum Einsatz. Diese Elektroheizer können in einem Gehäuse verbaut werden, welches zumindest eine Öffnung, insbesondere einen Schlitz, aufweist. Über diese Öffnung kann das im Volumen befindliche Medium und/oder Fluid zu den Elektroheizern geführt oder aus dem Volumen abgeführt werden. Vorzugsweise kann das Medium und/oder das Fluid durch die Öffnung in das Gehäuse vordringen, insbesondere eingesogen werden, und vom Heizelement des Elektroheizers erhitzt werden. Das erhitzte Medium und/oder Fluid kann in der Folge vom Ventilator des Elektroheizers wieder in das Volumen eingebracht werden. Hierbei kann ein Kreislauf entstehen, welcher zu einer besonders konstanten Temperierung des im Volumen befindlichen Mediums und damit einer besonders effizienten Inaktivierung der Mikroorganismen auf und/oder in den Objekten führt. Durch das Einbringen des Mediums über den Ventilator des Elektroheizers kann außerdem eine besonders effiziente Konvektion des Mediums innerhalb des Volumens erzielt werden. Bevorzugt ist der Ventilator als Axialventilator ausgebildet. Dadurch kann im Volumen eine gleichmäßige Wärmeverteilung sichergestellt werden.

Alternativ kann das Gehäuse auch eine Zufuhr und eine Abfuhr aufweisen, über welche das Medium Fluid im Volumen ausgetauscht werden kann. Dies bietet den Vorteil, dass der Atmosphäre des Volumens frisches Medium beigemengt werden kann. Hierbei dringt das Medium vom Volumen über die Öffnung in das Gehäuse und kann aus diesem über die Abfuhr abgeleitet werden. Über die gesonderte Zufuhr des Gehäuses kann, vorzugsweise zeitgleich, frisches Medium in das Gehäuse eingebracht, von der Heizwendel des Elektroheizers erhitzt und über den Ventilator in das Volumen befördert werden.

Gemäß einem, nicht zur Erfindung gehörenden Beispiel, kann der Infrarotstrahler das Medium und/oder das Fluid im Volumen durch ein Aussenden von Infrarotstrahlen, vorzugsweise direkt, erhitzen. Dabei unterliegen Medium und/oder Fluid im Volumen der natürlichen Konvektion. Um die Konvektion des Mediums und/oder Fluids und damit die Wärmeverteilung im Volumen zu erhöhen, können ein oder mehrere Ventilatoren vorgesehen sein. Auf diese Weise kann bei Verwendung von Infrarotstrahlern und/oder Infrarotplatten eine besonders gleichmäßige Wärmeverteilung erzielt werden. Zudem bieten Infrarotstrahler, insbesondere Infrarotplatten, den Vorteil, dass Wärme auf einfache Weise ins Innere des Objektes eingebracht und dort befindliche Mikroorganismen effizienter inaktiviert werden können. Die Verwendung von plattenförmigen Heizelementen, insbesondere in großflächiger Ausführung, kann eine besonders gleichmäßige Wärmeübertragung von den Heizelementen auf das Volumen sicherstellen. Im Falle von Infrarotplatten kann die Wärmeübertragung weitestgehend unter Ausschluss eines Übertragungsmediums stattfinden und im Volumen befindliche Objekte direkt an deren Oberfläche erwärmt werden. Elektroheizplatten können das im Volumen befindliche Medium und/oder Fluid zur Wärmeübertragung nutzen, sodass auch das im Volumen befindliche Medium und/oder Fluid eine prozessspezifische Temperatur aufweist. Um die Vorteile beider Heizelemente zu verbinden, können sowohl Elektroheizer als auch Infrarotstrahler im Volumen vorgesehen sein. Darüber hinaus können zusätzliche Heizelemente an einer dritten Seitenfläche und/oder vierten Seitenfläche und/oder der Deckfläche angeordnet sein.

Alternativ kann zusätzlich zu den Heizelementen zumindest ein UV-C Strahler zum Inaktivieren von Mikroorganismen vorgesehen sein. Hierbei wird durch die vom Strahler abgegebene UV-C Strahlung, welche gemäß DIN 5031-7 vom Jänner 1984 eine Wellenlänge von 280 nm bis 100 nm aufweist, eine besonders effiziente oberflächliche Inaktivierung erzielt werden. Entsprechend ist auch eine Kombination von Heizelementen und Strahlern möglich.

Es hat sich bewährt, dass eine Basis vorgesehen ist, welche die Grundfläche umfasst und zumindest eine Ausnehmung für ein Hebegerät, insbesondere einen Gabelstapler, aufweist. Die Ausnehmung ist so angeordnet, dass diese die Gabelfortsätze eines handelsüblichen Hebegerätes aufnehmen kann. Hierbei ist die Ausnehmung entweder als längliche Öffnung oder zum Untergrund hin geöffnet ausgebildet, wobei vorzugsweise jede Seite der Basis mit zwei Ausnehmungen versehen ist. Alternativ kann die Basis auch aus vier bis neun Standfüßen bestehen, welche auf der Unterseite der Grundfläche angebracht werden, wobei eine Verbindung zwischen der Grundfläche und den Standfüßen auf stoffschlüssige, formschlüssige und/oder kraftschlüssige Art erfolgen kann. Obgleich eine Anbringung durch Schrauben, Nieten, Kleben oder Nageln erfolgen kann, wird ein Anschweißen der Standfüße an der Grundfläche bevorzugt.

Mit Vorteil ist die Grundfläche des Volumens zur Aufnahme einer Flachpalette, bevorzugt einer nach EN 13698-1 genormten Palette, ausgebildet. Dies ermöglicht das zeitsparende Beschicken des Volumens, vorzugsweise mithilfe eines Hebegerätes, mit auf einer Flachpalette befindlichen Objekten. Vorzugsweise weist die Flachpalette die in der Norm EN 13698-1 vom Juli 2003 angegebenen Merkmale und Maße auf.

Um die Vorrichtung in einen kontinuierlichen Produktionsprozess einzugliedern, kann vorgesehen sein, dass das Volumen von zwei Seiten zugänglich ist. Objekte können dann kontinuierlich oder quasi-kontinuierlich mit einer Verweilzeit in der Vorrichtung durch diese transportiert werden, beispielsweise mit einer Fördereinrichtung wie einem Förderband. Hierbei sind Deckfläche und Grundfläche über zwei gegenüberliegende Seitenflächen miteinander verbunden, sodass Objekte von einer Seite in das Volumen eintreten und es auf der gegenüberliegenden Seite wieder verlassen können. Die im Volumen vor den Seitenflächen angebrachte Inaktivierungseinrichtung sorgt für den benötigten Wärmeeintrag und gewährleistet eine effiziente Inaktivierung von Mikroorganismen. Zum Erhöhen des Wärmeeintrags kann eine zusätzliche Inaktivierungseinrichtung im Volumen vor der Deckfläche angeordnet werden.

Bevorzugt ist eine Oberseite der Grundfläche zur Aufnahme eines Bandförderers ausgebildet. Eine entsprechende Aufnahme stellt sicher, dass eine erfindungsgemäße Vorrichtung nahtlos in einen Produktionsprozess eingegliedert werden kann, sodass in und/oder auf Produkten befindliche Mikroorganismen im Zuge eines Prozesses inaktiviert werden können.

Zweckmäßigerweise ist die Basis mit zumindest einer Lasche zur Aufnahme zumindest eines ortsfesten Zuganges versehen. Hierdurch wird die Höhendifferenz zwischen Untergrund und Grundfläche durch beispielsweise eine Rampe überbrückt, sodass sich eine erfindungsgemäße Vorrichtung auch zur Aufnahme von Ladungsträgern wie Transportwagen, rollbaren Plattformen oder rollbaren Paletten eignet. Diese können auf einfache Weise manuell oder maschinell über die Rampe in das Volumen geschoben werden. In einer bevorzugten Variante sind an der Basis zwei Laschen vorgesehen, sodass entweder für eine Rampe zwei Auflagepunkte oder für zwei Rampen jeweils ein Auflagepunkt bereitgestellt wird.

Es hat sich bewährt, dass die Lasche eine Öffnung zur Aufnahme von Befestigungsmitteln aufweist. Dabei kann die Öffnung zur formschlüssigen und/oder kraftschlüssigen Aufnahme von Befestigungsmitteln ausgebildet sein. Bevorzugt ist die Nutzung eines Bolzens als Befestigungsmittel. Alternativ kann jedoch auch eine Schraube verwendet werden, wofür ein entsprechendes Gewinde in der Öffnung vorgesehen ist.

Um einen Wärmeverlust zu minimieren, kann vorgesehen sein, dass das Volumen von einer Seite zugänglich und ein Verschluss wie eine Tür vorgesehen ist, mit dem diese Seite verschließbar ist. Die Tür bedeckt hierbei die einzige offene Seitenfläche und ist über zumindest ein Scharnier, vorzugsweise zwei Scharniere, an einer angrenzenden Seitenfläche befestigt. In bevorzugter Weise verfügt die Tür über einen Schließmechanismus, welcher mithilfe eines Hebels bedienbar ist, sodass die Tür fest verschlossen werden kann und nicht unbeabsichtigt geöffnet wird bzw. sich selbsttätig öffnet. Alternativ kann die Tür auch über eines oder mehrere Scharniere an der Deckfläche befestigt sein, wobei insbesondere zwischen zumindest einer angrenzenden Seitenfläche und der Tür eine Gasdruckfeder zur Unterstützung des Öffnungsprozesses und zum Offenhalten der Tür vorgesehen ist. In einer weiteren Alternative ist eine Schiebetüre vorgesehen.

Bevorzugt ist ein im Wesentlichen quaderförmiger Rahmen zur Aufnahme der Inaktivierungseinrichtung sowie der Teile für Grundfläche, Deckfläche und Seitenflächen vorgesehen. Die Hauptbestandteile des Rahmens, insbesondere Träger und Steher, sind vorzugsweise aus einem Metall, einer Legierung oder einem ähnlichen mechanisch bearbeitbaren Material gefertigt. Hierbei dient der Rahmen nicht ausschließlich dem Verbinden der einzelnen Bauteile, sondern erhöht zusätzlich auch die Stabilität der Vorrichtung. In bevorzugter Weise umfasst der Rahmen mehrere Träger, welche in einem geringen vertikalen Abstand zur Grundfläche eine Begrenzung, insbesondere mit den in der Norm EN 13698-1 vom Juli 2003 vorgeschriebenen Dimensionen, schaffen, sodass eine entsprechend genormte Flachpalette exakt in der Vorrichtung positioniert werden kann. Zudem kann durch ein Einspannen oder Einschieben der Inaktivierungseinrichtung zwischen zwei Rahmenbestandteile eine lösbare Befestigung erfolgen. Alternativ kann zur Befestigung der Inaktivierungseinrichtung am Rahmen auch eine formschlüssige Verbindung, insbesondere eine Schraubverbindung, vorgesehen sein. Die Grundfläche, Deckfläche und Seitenflächen werden durch eine formschlüssige Verbindung, insbesondere eine Schraubverbindung, lösbar am Rahmen angebracht. In einer alternativen Ausbildung können diese auch stoffschlüssig, insbesondere durch Schweißnähte oder Schweißpunkte, mit dem Rahmen verbunden sein.

In vorteilhafter Weise ist ein Rahmen aus einer Vielzahl von, miteinander stoffschlüssig und/oder kraftschlüssig verbundenen, Metallprofilen ausgebildet. Bevorzugt bestehen Metallprofile aus Metall-Halbzeug und werden zum Ausbilden des quaderförmigen Rahmens an deren Enden miteinander stoffschlüssig, insbesondere durch eine Schweißnaht oder Schweißpunkte, verbunden. Als Träger eingesetzte, horizontale Metallprofile zur Aufnahme von Inaktivierungseinrichtungen sind entweder einseitig oder an zwei gegenüberliegenden Seiten mit u-förmigen Fortsätzen ausgebildet.

Hierbei sind die Fortsätze mit dem Metallprofil stoffschlüssig, insbesondere durch eine Schweißverbindung, verbunden. Zur Aufnahme einer Inaktivierungseinrichtung zwischen zwei Metallprofilen werden die offenen Seiten der beiden, auf den Metallprofilen angebrachten, u-förmigen Fortsätze einander gegenübergestellt. Umfasst die Inaktivierungseinrichtung ein Gehäuse mit einem oder mehreren Elektroheizern und/oder Infrarotstrahlern, kann dieses Gehäuse an zumindest einem Metallprofil befestigt werden. Vorzugsweise wird das Gehäuse zwischen zwei Metallprofilen positioniert und an diesen, insbesondere formschlüssig und/oder stoffschlüssig, befestigt. Metallprofile, die zur Aufnahme von Grundfläche, Deckfläche oder Seitenflächen dienen, sind mit entsprechenden Bohrungen und/oder Gewinden versehen.

Um eine Gewichtsersparnis bei gleichbleibend hoher Stabilität zu erzielen, kann vorgesehen sein, dass die Metallprofile hohl ausgebildet sind und einen runden, quadratischen, rechteckigen oder vieleckigen Querschnitt aufweisen.

Es ist günstig, wenn Grundfläche, Deckfläche und Seitenflächen aus einem wärmedämmenden Material, bevorzugt aus mehreren Schichten, gebildet sind. Hierdurch wird weniger Energie zum Aufheizen des Volumens bzw. der Objekte im Inneren der Vorrichtung benötigt und ein Inaktivierungsprozess kann schneller abgeschlossen werden. Ein bevorzugter mehrschichtiger Aufbau umfasst zumindest eine wärmedämmende Schicht und eine dekorative äußere Schicht. Des Weiteren kann eine zusätzliche Schicht aus feuerfestem Material oder ein Kühlkörper, insbesondere um eine Fläche an einer Hinterseite eines Heizelementes zu kühlen, vorgesehen sein.

Um eine vollständige Inaktivierung von Mikroorganismen zu erzielen, hat es sich bewährt, dass eine Temperatur im Volumen mit der Inaktivierungseinrichtung auf zwischen 60 °C und 100 °C, bevorzugt zwischen 70 °C und 90 °C, besonders bevorzugt 80 °C, einstellbar ist. Die Verweildauer der Objekte im erwärmten Volumen hängt hierbei maßgeblich von der Art der zu inaktivierenden Mikroorganismen ab. Während viele Arten von Viren eine geringe Hitzeresistenz aufweisen und bereits bei Temperaturen zwischen 55 °C und 80 °C innerhalb weniger Minuten inaktiviert werden können, müssen sporenbildende Bakterien und Pilze für eine entsprechende Inaktivierung mehrere Stunden bei 100 °C oder mehr behandelt werden. Um eine Inaktivierung von sporenbildenden Bakterien und Pilzen sicherzustellen, kann die Verweildauer der Objekte in der Vorrichtung auf 180 Minuten oder mehr festgelegt werden.

Eine Verwendung einer erfindungsgemäßen Vorrichtung besteht darin, in und/oder auf einem oder mehreren Objekten befindliche Mikroorganismen, insbesondere Bakterien und/oder Viren, zu inaktivieren. Dementsprechend wird eine erfindungsgemäße Vorrichtung bevorzugt zu diesem Zweck verwendet.

Die verfahrensmäßige Aufgabe wird erfindungsgemäß dadurch gelöst, dass bei einem Verfahren der eingangs genannten Art das Volumen durch eine im Volumen angeordnete Inaktivierungseinrichtung beheizt wird.

Um das Verfahren im kontinuierlichen Betrieb nutzen zu können, kann vorgesehen sein, dass das Volumen von zwei Seiten zugänglich und die Oberseite der Grundfläche zur Aufnahme eines Bandförderers ausgebildet ist. Hierbei wird das Volumen kontinuierlich mit, auf dem Bandförderer befindlichen, Objekten beschickt, wobei auf und/oder in den Objekten befindliche Mikroorganismen während einer Durchfahrt inaktiviert werden. Eine Durchfahrtsgeschwindigkeit richtet sich deshalb nach den zu inaktivierenden Mikroorgansimen und kann entsprechend angepasst werden. Auch ein Halten in der Vorrichtung und somit ein quasi-kontinuierlicher Betrieb ist möglich.

Weitere Merkmale, Vorteile und Wirkungen der Erfindung ergeben sich aus den nachfolgend dargestellten Ausführungsbeispielen. Die Zeichnungen, auf welche Bezug genommen wird, zeigen:

Fig. 1 eine perspektivische Darstellung einer Ausführungsvariante der Vorrichtung;
Fig. 2 einen Schnitt entlang der Linie II-II gemäß Fig. 1;
Fig. 3 eine perspektivische Darstellung einer weiteren Ausführungsvariante der Vorrichtung;
Fig. 4 einen Schnitt entlang der Linie IV-IV gemäß Fig. 3;
Fig. 5 eine perspektivische Darstellung eines Rahmens der Vorrichtung gemäß Fig. 1;
Fig. 6 Querschnitte der Metallprofile des Rahmens;
Fig. 7 eine perspektivische Darstellung einer weiteren Ausführungsvariante der Vorrichtung;
Fig. 8 einen Schnitt entlang der Linie VIII-VIII gemäß Fig. 7;
Fig. 9 eine perspektivische Darstellung eines Rahmens der Vorrichtung gemäß Fig. 7;
Fig. 10 eine Frontansicht des Rahmens der Vorrichtung gemäß Fig. 7.

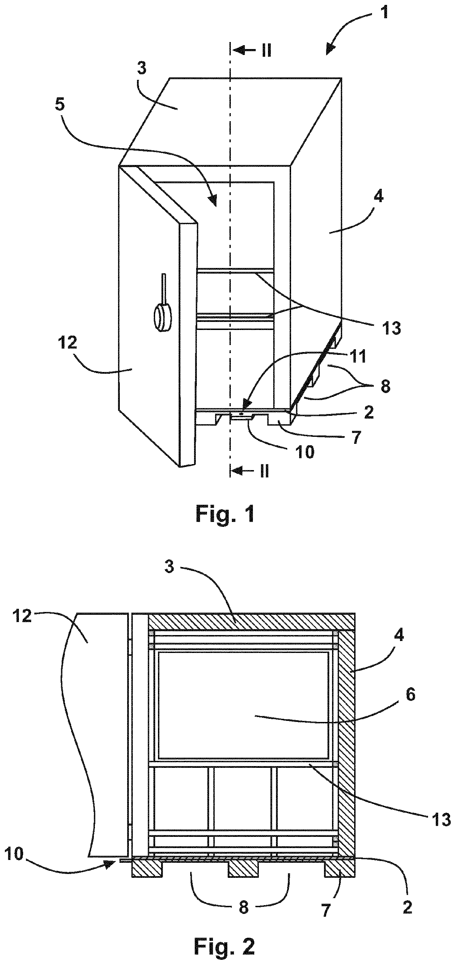

Fig. 1 zeigt eine perspektivische Darstellung einer ersten Ausführungsform einer Vorrichtung 1 zum Inaktivieren von Mikroorganismen, insbesondere von Bakterien und/oder Viren, wobei eine Grundfläche 2, eine Deckfläche 3 und zwei Seitenflächen 4 sichtbar sind. Zudem bilden die Flächen ein Volumen 5, welches von der Vorderseite zugänglich und durch eine Tür 12, hier mit einem Griff ausgeführt, verschließbar ist. Die Tür 12 ist an der linken Seite mit zwei Scharnieren an der Seitenfläche 4 befestigt, sodass diese nach vorne schwenkbar ist. Unter der Grundfläche 2 ist eine Basis 7 angeordnet, welche mit einer Lasche 10 samt Öffnung 11 sowie Ausnehmungen 8 zum Zusammenwirken mit einem Hebegerät, insbesondere einem Gabelstapler, ausgebildet ist. In dieser Ausführungsvariante weist die Basis 7 zwei Ausnehmungen 8 pro Seite auf, wobei alternativ auch nur eine oder zwei Ausnehmungen 8, welche sich von der Vorderseite zur Rückseite der Basis 7 erstrecken, vorgesehen sein können. Da die Grundfläche des Volumens 5 zur Aufnahme einer Flachpalette, insbesondere einer nach EN 13698-1 genormten Holzpalette, ausgebildet ist, kann ein Hebegerät zum Beschicken des Volumens 5 mit einer solchen Flachpalette genutzt werden. Alternativ kann ein ortsfester Zugang, insbesondere eine Rampe, an den Laschen 10 befestigt werden, sodass ein Ladungsträger in das Volumen 5 gerollt werden kann. Eine entsprechende Rampe kann in weiterer Folge durch Zusammenwirken eines Befestigungsmittels mit der Öffnung 11 fixiert werden. Ferner ist an der hinteren Seitenfläche 4 ein Teil des, von den Seitenflächen 4 und der Deckfläche 3 bedeckten Rahmens 13 erkennbar, welcher unter anderem zur Aufnahme von Heizelementen 6 dient.

In Fig. 2 ist ein Schnitt entlang der Linie II-II gemäß Fig. 1 gezeigt, anhand dessen der Aufbau des Rahmens 13 und der Basis 7 ersichtlich ist. Der Rahmen 13 besteht aus einer Vielzahl von Metallprofilen, wobei die beiden Träger nahe der Grundfläche 2 zur genauen Positionierung einer Flachpalette dienen. Des Weiteren ist auch die Positionierung eines Heizelementes 6 ersichtlich, welches zwischen zwei Trägern in der oberen Hälfte des Rahmens 13 positioniert ist. Alternativ wäre auch die Befestigung eines zusätzlichen Heizelementes 6 in der unteren Hälfte der Rahmens 13 möglich. Die Tür 12 ist mit zwei Scharnieren an der Seitenwand 4 befestigt, könnte alternativ aber mit nur einem Scharnier und/oder an der Deckfläche 3 befestigt sein.

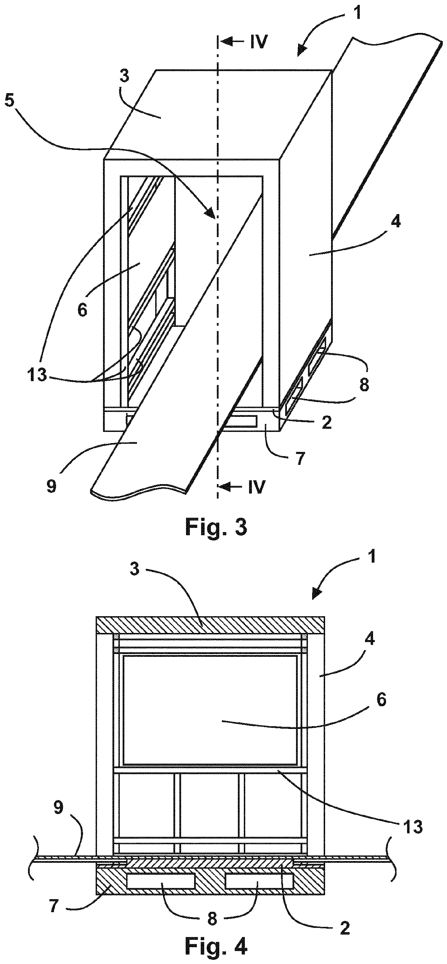

In Fig. 3 ist eine perspektivische Darstellung einer weiteren Ausführungsvariante gezeigt, welche zum kontinuierlichen Betrieb einer Vorrichtung 1 geeignet ist. Ähnlich Fig. 1 sind die Grundfläche 2, Deckfläche 3 und Seitenflächen 4 der Vorrichtung 1 erkennbar. Das Entfallen einer Tür 12 gewährt hier mehr Einblick ins Volumen 5 der Vorrichtung 1, sodass ein Heizelement 6 und ein Teil des Rahmens 13 ersichtlich sind. Eine Vorrichtung 1 dieser Ausführungsvariante verfügt zum kontinuierlichen Betrieb über eine Aufnahme für einen Bandförderer 9. Die Aufnahme erfolgt über die spezielle Ausbildung der dem Volumen 5 zugewandten Seite der Grundfläche 2. Eine Basis 7 ist hierbei an der Unterseite der Grundfläche 2 angeordnet und mit zwei länglichen Ausnehmungen 8 pro Seite versehen, welche zum Untergrund hin zwar nicht geöffnet ausgebildet sind, aber dennoch einen Transport der Vorrichtung 1 mittels Hebegerät ermöglichen. Da eine manuelle Beschickung des Volumens 5 in dieser Variante nicht vorgesehen ist, entfallen auch die Laschen 10 zur Anbringung eines ortsfesten Zuganges.

Fig. 4 zeigt einen Schnitt entlang der Linie IV-IV gemäß Fig. 3, wobei eine spezielle Ausführung der Grundfläche 2 eindeutig erkennbar ist. Im Weiteren ist ersichtlich, dass die Ausnehmungen 8 zum Untergrund hin geschlossen ausgeführt sind und die Basis 7 im Wesentlichen mit dem Querschnitt einer handelsüblichen Flachpalette ident ist. Alternativ zur ausschließlichen Anbringung des Heizelementes 6 in der oberen Hälfte des Rahmens 13 kann ein zusätzliches Heizelement 6 in der unteren Hälfte angebracht werden.

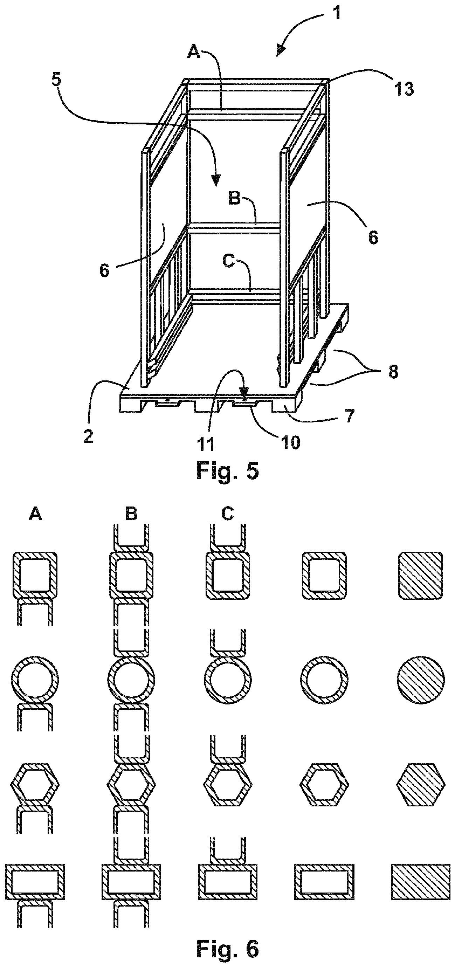

In Fig. 5 ist eine perspektivische Darstellung des Rahmens 13 einer Vorrichtung 1 gemäß Fig. 1 gezeigt. Der aus einer Vielzahl von Metallprofilen bestehende Rahmen 13 wird hierbei auf der Grundfläche 2 angebracht und bietet eine Reihe von Befestigungspunkten für die Deckfläche 3 und die Seitenflächen 4. In dieser Ausführungsvariante sind vor den Seitenflächen 4 zwei Heizelemente 6 angeordnet, wobei diese von jeweils zwei Metallprofilen des Rahmens 13 aufgenommen werden. Hierzu weisen einige Metallprofile die besonderen Querschnitte A, B und C auf. In geringem Abstand zur Grundfläche 2 sind an den Seitenteilen des Rahmens 13 jeweils zwei Metallprofile, insbesondere in horizontaler Ausrichtung und übereinander, angeordnet, welche zur passgenauen Aufnahme und damit exakten Positionierung einer Flachpalette, insbesondere einer nach EN 13698-1 genormten Holzpalette, im Volumen 5 dienen. Alternativ kann zu diesem Zweck anstatt von zwei Metallprofilen auch nur ein Metallprofil pro Seitenteil vorgesehen sein.

In Fig. 6 sind die Querschnitte der Metallprofile eines Rahmens 13 gemäß Fig. 5 dargestellt. Hierbei sind die unterschiedlichen Grundformen der Querschnitte gezeigt, welche allesamt als Hohlprofile ausgebildet sind. An den Unter- bzw. Oberseiten der in Fig. 5 indizierten Metallprofile A, B und C werden, vorzugsweise durch stoffschlüssige Verbindungen wie Schweißnähte oder Schweißpunkte, u-förmige Profile angebracht. Diese dienen zur Aufnahme von Heizelementen 6, welche zwischen den Profilen eingespannt oder in diese eingeschoben werden. Die Querschnitte der in Fig. 5 gezeigten Metallprofile A, B und C gelten exemplarisch für sämtliche in derselben horizontalen Ebene verbauten Metallprofile.

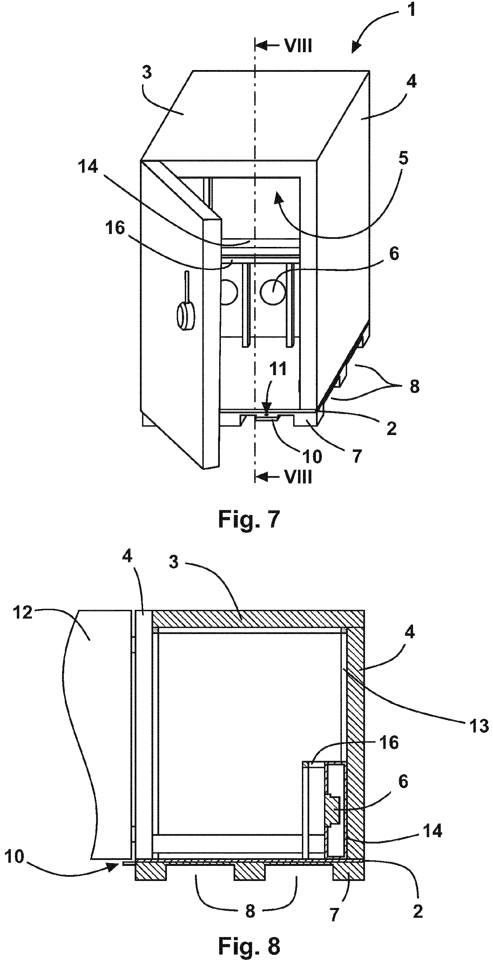

In Fig. 7 ist eine perspektivische Darstellung einer weiteren Ausführungsvariante der Vorrichtung 1 gezeigt. Hierbei sind ebenfalls die Grundfläche 2, die Deckfläche 3 und zwei Seitenflächen 4 erkennbar. Die Flächen bilden das Volumen 5, welches von der Vorderseite durch die Tür 12 mit Griff zugänglich ist. Die Tür 12 ist an der linken Seite mit zwei Scharnieren, in dieser Darstellung nicht erkennbar, an der Seitenfläche 4 befestigt und nach vorne schwenkbar. An der Grundfläche 2 ist eine Basis 7 angeordnet, welche mit zwei Laschen 10 samt Öffnungen 11 zum Befestigen zumindest einer Rampe ausgebildet ist. Darüber hinaus weist die Basis 7 zwei Ausnehmungen 8 pro Seite auf, welche zum Zusammenwirken mit einem Hebegerät, insbesondere einem Gabelstapler, ausgebildet sind. Die Grundfläche 2 weist eine Fläche auf, die groß genug ist, um die Flachpalette, insbesondere eine nach EN 13698-1 genormte Holzpalette, aufzunehmen. Folglich kann das Hebegerät zum Beschicken des Volumens 5 mit einer solchen Flachpalette genutzt werden. Alternativ kann die Rampe über Befestigungsmittel an den Laschen ortsfest an der Basis 7 angebracht werden, sodass ein Ladungsträger in das Volumen 5 gerollt werden kann. An der hinteren Seitenfläche 4 ist ein Teil des Rahmens 13 erkennbar, an welchem die Seitenflächen 4, die Deckfläche 3 und die Grundfläche 2 befestigt sind. Zudem ist ein Gehäuse 14 erkennbar, welches an den Seiten ebenfalls am Rahmen 13 befestigt ist. Innerhalb dieses Gehäuses 14 sind die Heizelemente 6 angeordnet, welche bevorzugt als Elektroheizer ausgebildet sind und Ventilatoren aufweisen. Diese Ventilatoren können durch eine bevorzugt rund ausgebildete Öffnung von den Heizelementen 6 erzeugte Wärme in das Volumen 5 einbringen. Um einen ausreichenden Abstand zwischen Gehäuse 14, insbesondere den Ventilatoren der Heizelemente 6, und den im Volumen 5 befindlichen Objekten sicherzustellen, ist ein Schutzrahmen 16 vorgesehen. Dieser ist vorzugsweise aus Metallprofilen gebildet und wirkt beim Einbringen von Objekten in das Volumen 5 als Begrenzer.

Fig. 8 zeigt einen Schnitt entlang der Linie VIII-VIII gemäß Fig. 7, anhand welchem der Aufbau des Rahmens 13, der Basis 7 samt Ausnehmungen 8 und Lasche 10, des Gehäuses 14 und des Heizelementes 6 ersichtlich ist. Der Rahmen 13 besteht auch in dieser Ausführungsvariante aus einer Vielzahl von Metallprofilen, wobei jedoch nur ein Träger nahe der Grundfläche 2 zur genauen Positionierung der Flachpalette vorgesehen ist. Zudem kann dieser Darstellung auf eindeutige Weise entnommen werden, wie weit der Schutzrahmen 16 und das Gehäuse 14 in das Volumen 5 vorstehen. Der Schutzrahmen 16 kann auch knapper am Gehäuse 14 positioniert werden, ohne die Funktion des Heizelementes 6 maßgeblich zu beeinträchtigen. Das Heizelement 6 ist innerhalb des Gehäuses 14 angeordnet und ist als Elektroheizer ausgebildet, umfasst also den Ventilator und die Heizwendel. Die Tür 12 ist mit zwei Scharnieren an der Seitenwand 4 befestigt, könnte alternativ aber mit nur einem Scharnier und/oder an der Deckfläche 3 befestigt sein.

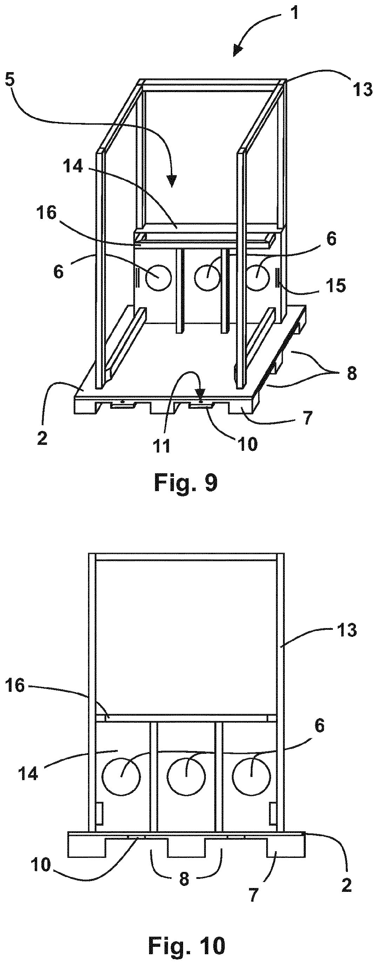

In Fig. 9 ist eine perspektivische Darstellung eines Rahmens 13 der Vorrichtung 1 gemäß Fig. 7 gezeigt. Der aus einer Vielzahl von Metallprofilen bestehende Rahmen 13 wird auch in dieser Ausführungsvariante auf der Grundfläche 2 angebracht. Dabei bietet der Rahmen 13 eine Reihe von Befestigungspunkten für die Deckfläche 3, die Seitenflächen 4 sowie das Gehäuse 14. Im Gehäuse 14 sind drei Heizelemente 6 angeordnet und in einem oberen Bereich des Gehäuses 14 ist der Schutzrahmen 16 angebracht. Zudem kann dieser Darstellung eine Anordnung von Öffnungen, insbesondere Schlitzen 15, des Gehäuses 14 entnommen werden, durch welche im Volumen 5 befindliches Medium in das Gehäuse 14 eindringen kann. Um ein Blockieren der Schlitze 15 zu verhindern, sind diese in einer Ebene mit Stehern des Rahmens angeordnet. Das über die Schlitze 15 in das Gehäuse 14 eindringende Medium wird von den Heizwendeln der Heizelemente 6 erhitzt und anschließend durch die Ventilatoren, insbesondere Axialventilatoren, der Heizelemente 6 wieder zurück in das Volumen 5 befördert. In dieser Ausbildungsvariante sind die Querschnitte der Metallprofile des Rahmens 13 von geringerer Relevanz, weshalb diese sämtliche in der Fig. 6 angegebenen Querschnitte aufweisen können. In geringem Abstand zur Grundfläche 2 ist an den Seitenteilen des Rahmens 13 jeweils ein Metallprofil in horizontaler Ausrichtung angeordnet, welches zur passgenauen Aufnahme und damit exakten Positionierung einer Flachpalette, insbesondere einer nach EN 13698-1 genormten Holzpalette, im Volumen 5 dient. Der Schutzrahmen 16 begrenzt eine Tiefe beim Einschieben der Flachpalette in das Volumen 5.

In Fig. 10 ist eine Frontansicht des Rahmens 13 der Vorrichtung 1 gemäß Fig. 9 gezeigt. Hierbei kann der Abstand zwischen den Heizelementen 6 und die Positionierung des Gehäuses 14 im Volumen 5 auf einfache Weise erkannt werden. Ebenso ist ersichtlich, dass die Schlitze 15 in einer Ebene mit vertikalen Stehern des Rahmens 13 liegen und deshalb nicht zu sehen sind. Die Positionierungen der Metallprofile zur seitlichen Begrenzung der Fläche für die Flachpalette sowie des Schutzrahmens 16 zur Begrenzung der Tiefe beim Einschieben der Flachpalette sind aus dieser Perspektive gut erkennbar.

Nachstehend sind Versuchsreihen zur bakteriziden und viruziden Wirksamkeit der Ausführungsvariante der Vorrichtung 1 gemäß Fig. 7 gezeigt.

**Versuchsreihe bakterizide Wirksamkeit einer Ausführungsvariante einer Vorrichtung 1**

Die getestete Ausführungsvariante der Vorrichtung 1 sieht als Inaktivierungseinrichtung drei Elektroheizer und eine Sprühnebelanlage vor, sodass im Inneren eines Volumens 5 eine Atmosphäre aus Umgebungsluft und Wasserdampf, insbesondere feuchte Hitze, erzeugt wird. Hierzu wird demineralisiertes Wasser über die Sprühnebelanlage in Form eines Aerosols in das Volumen 5 eingebracht und durch die von den Elektroheizern eingebrachte Wärme verdampft.

Die Prüfkörper bestehen aus runden Edelstahlplättchen, Edelstahl 1.4301 nach EN 10088-1 vom Dezember 2014, mit einem Durchmesser von 40 Millimeter (mm) und einer Dicke von 1,5 mm, welche mit einer Prüfsuspension kontaminiert werden. Eine derartige Prüfsuspension setzt sich zu neun Teilen aus einer Keimsuspension, welche aus einem Prüfkeim *Enterococcus faecium* (ATCC 6057) besteht und gemäß SOP 05-002 mit einer Verdünnungsflüssigkeit, bestehend aus 0,1 % Trypton und 0,85 % Natriumchlorid (NaCl), photometrisch auf eine Keimbelastung von $1,5{\times}10^9$ und $5{\times}10^9$ koloniebildende Einheiten (KBE) pro Milliliter (ml) eingestellt wird, und aus einem Teil, 0,3 Gramm (g) pro Liter (l), einer Belastungssubstanz Rinderserumalbumin zusammen.

Zum Kontaminieren der Prüfkörper werden 0,05 ml der Prüfsuspension auf einer Fläche von 3 Quadratzentimeter ($cm^2$) ($\pm$ 0,5 $cm^2$) des Prüfkörpers verteilt. Kontaminierte Prüfkörper werden für maximal 90 Minuten (min) bei 37 °C im Brutschrank getrocknet.

Im Anschluss werden die Prüfkörper auf etwa halber Höhe des Volumens 5, zwischen Grundfläche 2 und Deckfläche 3, eingebracht. Diese werden in einem vorderen Bereich nahe einer Tür 12, einem mittleren Bereich in halber Tiefe zwischen Tür 12 und der hinteren Seitenwand 4 sowie in einem hinteren Bereich nahe der hinteren Seitenwand 4 positioniert. Die Vorrichtung 1 wird in Betrieb genommen und die Inaktivierung der im Volumen 5 befindlichen Mikroorganismen über einen Zeitraum von 60 min durchgeführt. Dabei wird die Zeit ab Erreichen einer Temperatur von 80 °C gemessen.

Die Prüfkörper werden nach einem Inaktivierungsvorgang unter Zuhilfenahme eines Bechers, mehrerer Glaskugeln und 10 ml Elutionslösung, bestehend aus 0,1 % Trypton und 0,85 % Natriumchlorid (NaCl), mit der kontaminierten Seite zum Becherboden für 10 min auf einem Rotationsschüttler bei 150 Umdrehungen pro Minute eluiert.

Derart eluierten Proben werden in Doppelbestimmung mit einem Nährmedium aus Caseinpepton-Sojabohnenmehlpepton-Agar (CSA) im Plattengussverfahren untersucht. Hierzu werden jeweils 1,0 ml der eluierten Probe direkt, einer $10^{-1}$ Verdünnung und einer $10^{-2}$ Verdünnung entnommen. Ein Rest der Elutionslösung wird membranfiltriert und der Filter (MicroPlus-31 STL Membrane Filters; Lot: A29511376) auf einer CSA Platte exponiert. Der Prüfkörper wird nach der Membranfiltration entnommen und mit CSA übergossen. Anschließend werden die Proben samt Nährmedium bei 36 °C für 48 Stunden (h) inkubiert.

Der Reduktionsfaktor berechnet sich durch Subtraktion des Logarithmus der KBE des Prüfkörpers vom Logarithmus der KBE bei Kontrolle nach Einwirkzeit einer Trocknungskontrolle. Bei dieser Trocknungskontrolle wird ein Keimträger je Keim- bzw. Belastungskombination nach einer Trocknung für die Dauer des Prozesses im Labor gelagert und anschließend zusammen mit den Prüfkörpern aus dem Hauptversuch analysiert. Die Elution des Keimträgers der Trocknungskontrolle erfolgt analog zu jener der Prüfkörper.

Zusätzlich zur Trocknungskontrolle werden auch andere Positivkontrollen durchgeführt. Hierzu werden von der Prüfkeimsuspension zwei Verdünnungen von $10^{-6}$ und $10^{-7}$ erstellt und jeweils 1,0 ml in Doppelbestimmung membranfiltriert und der Filter auf CSA überführt. Diese Positivkontrolle wird doppelt durchgeführt und Prüfkeimsuspension N1 bzw. N2 genannt. Darüber hinaus wird eine Anschmutzkontrolle, genannt Anschmutzkontrolle Ko, durchgeführt, wobei ein Keimträger je Keim- bzw. Belastungskombination direkt nach der Trocknung analysiert wird. Die Elution dieser Keimträger erfolgt analog zu jener der Prüfkörper. Mit Verdünnungsflüssigkeit werden Verdünnungen von $10^{-4}$ und $10^{-5}$ hergestellt und 1,0 ml in Doppelbestimmung mit CSA übergossen.

Tabellen 1, 2 und 3 zeigen Ergebnisse der bakteriziden Wirksamkeit der Vorrichtung 1 anhand von drei Prüfläufen. Prüflauf 3 wurde analog zu den Prüfläufen 1 und 2, jedoch an einem anderen Tag durchgeführt.

Tabelle 1: Bakterizide Wirksamkeit Prüflauf 1

| Zeit [min] | Prüfkörper | Position | KBE/Prüfkörper | KBE/Prüfkörper [log] | Reduktionsfaktor [log] |
|---|---|---|---|---|---|
| 60 | 1 | vorne | 0 | 0 | ≥6,93 |
| | 2 | mitte | 0 | 0 | ≥6,93 |
| | 3 | hinten | 0 | 0 | ≥6,93 |

Tabelle 2: Bakterizide Wirksamkeit Prüflauf 2

| Zeit [min] | Prüfkörper | Position | KBE/Prüfkörper | KBE/Prüfkörper [log] | Reduktionsfaktor [log] |
|---|---|---|---|---|---|
| 60 | 1 | vorne | 0 | 0 | ≥6,93 |
| | 2 | mitte | 0 | 0 | ≥6,93 |
| | 3 | hinten | 0 | 0 | ≥6,93 |

Tabelle 3: Bakterizide Wirksamkeit Prüflauf 3

| Zeit [min] | Prüfkörper | Position | KBE/Prüfkörper | KBE/Prüfkörper [log] | Reduktionsfaktor [log] |
|---|---|---|---|---|---|
| 60 | 1 | vorne | 0 | 0 | ≥6,90 |
| | 2 | mitte | 0 | 0 | ≥6,90 |
| | 3 | hinten | 0 | 0 | ≥6,90 |

Darüber hinaus werden Validierungskontrollen vorgenommen, um den Einfluss der Elutionsflüssigkeit, genannt inhibitorischer Effekt des Mediums n1, der Membranfiltration, genannt inhibitorischer Effekt der Membranfiltration n2, und des Keimträgers, genannt Carrier-vermittelter inhibitorischer Effekt im Medium n3, auf das Analyseergebnis zu ermitteln.

Zur Untersuchung des inhibitorischen Effekts des Mediums n1 werden Prüfkörper mit Belastungssubstanz, Rinderserumalbumin, angeschmutzt und dem Prozess ausgesetzt. Die Prüfkörper werden nach Prozessende eluiert und 1,0 ml der Elutionsflüssigkeit und 1,0 ml der in Verdünnungsflüssigkeit $10^{-6}$ bzw. $10^{-7}$ verdünnten Bakteriensuspension in eine Petrischale gegeben und in Doppelbestimmung mit CSA übergossen und gemischt.

Um den inhibitorischen Effekt der Membranfiltration n2 zu untersuchen, werden Prüfkörper mit Belastungssubstanz, Rinderserumalbumin, angeschmutzt und dem Prozess ausgesetzt. Die Prüfkörper werden nach Prozessende eluiert und die erhaltene Elutionsflüssigkeit in Doppelbestimmung membranfiltriert. Anschließend wird ohne Filtertausch 1,0 ml der in Verdünnungsflüssigkeit $10^{-6}$ bzw. $10^{-7}$ verdünnten Bakteriensuspension in 50 ml Spülflüssigkeit membranfiltriert.

Der carrier-vermittelte inhibitorische Effekt im Medium n3 wird durch Anschmutzen der Prüfkörper mit Belastungssubstanz, Rinderserumalbumin, und Durchführen des Prozesses ermittelt. Nach dem Prozess werden die Prüfkörper in Petrischalen gelegt und zusammen mit 1,0 ml der in Verdünnungsflüssigkeit $10^{-6}$ bzw. $10^{-7}$ verdünnten Bakteriensuspension mit CSA übergossen.

Tabellen 4, 5 und 6 führen die Ergebnisse der Positiv- und Validierungskontrollen der Prüfläufe 1 und 2 an. Tabellen 7, 8 und 9 jene des Prüflaufes 3.

Tabelle 4: Positivkontrollen der Prüfkeimsuspension (Prüfläufe 1 und 2)

| Prüfkeimsuspension N1 Plattenguss | | | Prüfkeimsuspension N2 Membranfiltration | | |
|---|---|---|---|---|---|
| | Vc1 | Vc2 | | Vc1 | Vc2 |
| $10^{-6}$ | >330 | >330 | $10^{-6}$ | >165 | >165 |
| $10^{-7}$ | 35 | 40 | $10^{-7}$ | 45 | 42 |
| $\bar{x}$ log = 8,57 | | | $\bar{x}$ log = 8,64 | | |
| $7{,}70 \leq \log N1 \leq 9{,}70$ valide: ja | | | $7{,}70 \leq \log N2 \leq 9{,}70$ valide: ja | | |

Tabelle 5: Validierungskontrollen inhibitorische Effekte (Prüfläufe 1 und 2)

| Inhibitorischer Effekt des Mediums n1 | | | Inhibitorischer Effekt der Membranfiltration n2 | | | Carrier-vermittelter inhibitorischer Effekt im Medium n3 | | |
|---|---|---|---|---|---|---|---|---|
| | C1 | C2 | | C1 | C2 | | C1 | C2 |
| $10^{-6}$ | >330 | >330 | $10^{-6}$ | >165 | >165 | $10^{-6}$ | >330 | >330 |
| $10^{-7}$ | 46 | | $10^{-7}$ | 43 | 44 | $10^{-7}$ | 32 | 34 |
| $\bar{x}$ log = 8,62 | | | $\bar{x}$ log = 8,64 | | | $\bar{x}$ log = 8,27 | | |
| n1 > 0,5 N1 valide: ja | | | n2 > 0,5 N2 valide: ja | | | n3 > 0,5 N1 valide: ja | | |

Tabelle 6: Positivkontrollen Anschmutzung und Trocknung (Prüfläufe 1 und 2)

| Anschmutzungskontrolle Ko | | | | | Trocknungskontrolle T | | | | |
|---|---|---|---|---|---|---|---|---|---|
| | C1 | | C2 | | | C1 | | C2 | |
| $10^{-4}$ | >330 | >330 | >330 | >330 | $10^{-4}$ | >330 | >330 | >330 | >330 |
| $10^{-5}$ | 80 | 88 | 82 | 86 | $10^{-5}$ | 90 | 86 | 84 | 84 |
| $\bar{x}$ log = 6,92 | | | | | $\bar{x}$ log = 6,93 | | | | |
| | | | | | T > 6, valide: ja | | | | |

Tabelle 7: Positivkontrollen der Prüfkeimsuspension (Prüflauf 3)

| Prüfkeimsuspension N1 Plattenguss | | | Prüfkeimsuspension N2 Membranfiltration | | |
|---|---|---|---|---|---|
| | Vc1 | Vc2 | | Vc1 | Vc2 |
| $10^{-6}$ | >330 | >330 | $10^{-6}$ | >165 | >165 |
| $10^{-7}$ | 37 | 39 | $10^{-7}$ | 44 | 40 |
| $\bar{x} \log = 8,58$ | | | $\bar{x} \log = 8,62$ | | |
| $7,70 \leq \log N1 \leq 9,70$ valide: ja | | | $7,70 \leq \log N2 \leq 9,70$ valide: ja | | |

Tabelle 8: Validierungskontrollen inhibitorische Effekte (Prüflauf 3)

| Inhibitorischer Effekt des Mediums n1 | | | Inhibitorischer Effekt der Membranfiltration n2 | | | Carrier-vermittelter inhibitorischer Effekt im Medium n3 | | |
|---|---|---|---|---|---|---|---|---|
| | C1 | C2 | | C1 | C2 | | C1 | C2 |
| $10^{-6}$ | >330 | >330 | $10^{-6}$ | >165 | >165 | $10^{-6}$ | >330 | >330 |
| $10^{-7}$ | 35 | 40 | $10^{-7}$ | 36 | 41 | $10^{-7}$ | 39 | 36 |
| $\bar{x} \log = 8,57$ | | | $\bar{x} \log = 8,59$ | | | $\bar{x} \log = 8,57$ | | |
| n1 > 0,5 N1 valide: ja | | | n2 > 0,5 N2 valide: ja | | | n3 > 0,5 N1 valide: ja | | |

Tabelle 9: Positivkontrollen Anschmutzung und Trocknung (Prüflauf 3)

| Anschmutzungskontrolle Ko | | | | | Trocknungskontrolle T | | | | |
|---|---|---|---|---|---|---|---|---|---|
| | C1 | | C2 | | | C1 | | C2 | |
| $10^{-4}$ | >330 | >330 | >330 | >330 | $10^{-4}$ | >330 | >330 | >330 | >330 |
| $10^{-5}$ | 82 | 84 | 86 | 84 | $10^{-5}$ | 72 | 80 | 78 | 86 |
| $\bar{x} \log = 6,93$ | | | | | $\bar{x} \log = 6,90$ | | | | |
| | | | | | T > 6, valide: ja | | | | |

Entsprechend der vorstehenden Prüfergebnisse kann mit der angegebenen Ausführungsvariante der Vorrichtung 1 über eine Dauer von 60 min bei 80 °C feuchter Hitze unter niedriger Belastung eine ausreichende bakterizide Wirkung ($\geq$ 5 log) gegen den Prüfkeim *Enterococcus faecium* erzielt werden.

**Versuchsreihe viruzide Wirksamkeit einer Ausführungsvariante der Vorrichtung 1**

Der Versuchsaufbau, die Kontaminationsmethode und die Elution erfolgen analog zur Versuchsreihe zur bakteriziden Wirksamkeit der Vorrichtung 1.

Zur Herstellung der Virussuspension wird bovines Coronavirus, Stamm "S379 Riems" (RVB-0020) in der Zelllinie PT (CCLV-RIE 0011) vermehrt. Nach einem Einfrier- und Auftauprozess werden die Zelltrümmer durch niedertourige Zentrifugation abgetrennt. Der Überstand wird als Virussuspension eingesetzt. Die Prüfsuspension wird erhalten, indem neun Teile dieser Virussuspension mit einem Teil, 0,3 g/l, der Belastungssubstanz Rinderserumalbumin versetzt werden. Die Kontamination der Prüfkörper erfolgt analog zur Versuchsreiche zur bakteriziden Wirksamkeit und die kontaminierten Prüfkörper werden bis zur optischen Trockenheit, jedoch maximal 90 min, bei Raumtemperatur getrocknet.

Im Unterschied zur Elution der Versuchsreiche zur bakteriziden Wirksamkeit der Vorrichtung 1, kommt hier jedoch Dulbecco's Modified Eagle's Medium (DMEM) low Glucose und 2 % Fetales Kälberserum (FBS) als Elutionslösung zur Anwendung. Die Kultivierung der Elutionslösung und Prüfkörper im Hauptversuch erfolgt durch Verdünnung bis $10^{-8}$ in eiskaltem DMEM mit 2 % FBS und anschließender Titration auf einer Zellkultur. Hierzu werden die Proben in ganzzahligen Potenzen von 10 in 4 °C kaltem Medium (DMEM mit 2 % FBS) verdünnt und jeweils 100 Mikroliter ($\mu$l) einer

Verdünnung werden in 8 Vertiefungen einer 96-well-Mikrotiterplatte (MTP) mit permissiven Zellen gegeben. Die Prüfung erfolgt in dreifach-Bestimmung. Nach einer Inkubation von 7 bis 14 Tagen bei 37 °C in einem $CO_2$-Brutschrank werden mittels eines inversen Mikroskopes cytopathogene Effekte abgelesen. Der Reduktionsfaktor ergibt sich aus Subtraktion des Logarithmus des Infektionstiters ($TCID_{50}$) des Prüfkörpers vom Logarithmus des $TCID_{50}$ der Trocknungskontrolle, wobei der Infektionstiter die infektiöse Dosis darstellt.

Der $TCID_{50}$ wird nach der Methode von Spearman und Kärber berechnet:

$$m = x_k + \frac{d}{2} - d \sum p_i$$

Dabei ist m der Logarithmus des Titers bezogen auf das Testvolumen, $x_k$ der Logarithmus der kleinsten Verdünnungsstufe, bei welcher alle Testobjekte positiv reagieren, d der Logarithmus des Verdünnungsfaktors und $p_i$ die beobachtete Reaktionsrate.

Eine Berechnung des viruziden Effektes, welcher auch als logarithmischer Reduktionsfaktor der Infektiosität bezeichnet werden kann, erfolgt als Differenz der Viruskontrolle nach Antrocknung und den cytopathischen Resttitern nach Einwirkung der Prüfsuspension bei den jeweiligen Einwirkzeiten und wird als logarithmischer Reduktionsfaktor angegeben. Dabei ergibt sich der mittlere Reduktionsfaktor ($RF_{Ca}$) aus der Differenz des logarithmierten Virustiters aus den sechs Einzelbestimmungen der Viruskontrolle nach Antrocknung (Titer $T_{Ko}$) und den sechs Einzelbestimmungen des Prüfproduktes nach der jeweiligen Einwirkzeit (Titer $T_{RV}$).

$$T_{Ko} = \frac{c1 + c2 + c3 + c4 + c5 + c6}{6}$$

$$T_{RV} = \frac{c1 + c2 + c3 + c4 + c5 + c6}{6}$$

Dabei ist $T_{Ko}$ der mittlere, logarithmierte Titer der sechs Prüfkörper der Trocknungskontrolle, $T_{RV}$ der mittlere, logarithmierte Titer der sechs Prüfkörper aus dem Ansatz mit der Prüfsuspension und c eine Anzahl plaqueformender Einheiten (PFU) auf allen Platten einer Verdünnungsstufe.

Der mittlere RF der Prüfkörperversuche ($RF_{Ca}$) und sein 95 % Konfidenzintervall (KI) wird wie folgt berechnet:

$$RF_{Ca} = T_{Ko} - T_{RV}$$

$$S_m = \sqrt{d^2 \sum [p_i \times (1 - p_i) \div (n-1)]}$$

Dabei ist $S_m$ die Standardabweichung des logarithmierten Titers, d der Logarithmus des Verdünnungsfaktors, $p_i$ die beobachtete Reaktionsrate und n die Anzahl der Prüfobjekte je Verdünnung.

$$KI = \sqrt{(2S_{m(Kontrolle)})^2 + (2S_{m(Resttiter)})^2}$$

Sofern im Testansatz nach dem Inaktivieren mit der Vorrichtung 1 kein "Restvirus" mehr nachweisbar ist, entspricht das 95 % KI des Reduktionsfaktors des Testansatzes dem des Kontrollansatzes.

Von einer Wirksamkeit der Vorrichtung 1 gegen Viren kann immer dann ausgegangen werden, wenn der Reduktionsfaktor $\geq$ 4 $\log_{10}$-Stufen ist.

Wenn die cytotoxischen Effekte (CT) die cytopathischen Effekte (CPE) übersteigen, werden die errechneten Reduktionsfaktoren mit "$\geq$" ausgewiesen. Die Inaktivierung der Viren erfolgt, analog zur Versuchsreihe zur bakteriziden Wirksamkeit, über 60 min bei 80 °C unter feuchter Hitze im Volumen 5 der Vorrichtung 1.

Tabellen 10, 11 und 12 geben drei Prüfläufe der viruziden Wirksamkeit der Vorrichtung 1 an.

Tabelle 10: Viruzide Wirksamkeit Prüflauf 1

| Zeit [min] | Prüfkörper | Position | Virustiter des "Restvirus"/Prüfkörper [lg-TCID$_{50}$] | Reduktionsfaktor [log] | Mittelwert | ≥4 log |
|---|---|---|---|---|---|---|
| 60 | 1 | vorne | ≤2,50 | ≥4,19 | ≥4,19 | ja |
| | 2 | mitte | ≤2,50 | ≥4,19 | | |
| | 3 | hinten | ≤2,50 | ≥4,19 | | |

Tabelle 11: Viruzide Wirksamkeit Prüflauf 2

| Zeit [min] | Prüfkörper | Position | Virustiter des "Restvirus"/Prüfkörper [lg-TCID$_{50}$] | Reduktionsfaktor [log] | Mittelwert | ≥4 log |
|---|---|---|---|---|---|---|
| 60 | 1 | vorne | ≤2,50 | ≥4,19 | ≥4,06 | ja |
| | 2 | mitte | ≤2,50 | ≥4,19 | | |
| | 3 | hinten | 2,88 | 3,81 | | |

Tabelle 12: Viruzide Wirksamkeit Prüflauf 3

| Zeit [min] | Prüfkörper | Position | Virustiter des "Restvirus"/Prüfkörper [lg-TCID$_{50}$] | Reduktionsfaktor [log] | Mittelwert | ≥4 log |
|---|---|---|---|---|---|---|
| 60 | 1 | vorne | ≤2,50 | ≥4,25 | ≥4,08 | ja |
| | 2 | mitte | ≤2,50 | ≥4,25 | | |
| | 3 | hinten | 3,00 | 3,75 | | |

Darüber hinaus werden Positivkontrollen in Form einer Anschmutzungskontrolle K0 sowie einer Trocknungskontrolle T durchgeführt. Zur Anschmutzungskontrolle werden zwei Virus-Prüfkörper direkt nach der Trocknung analysiert. Die Elution erfolgt wie oben beschrieben und es werden mit DMEM mit 2 % FBS Verdünnungen bis $10^{-8}$ hergestellt, wobei die einzelnen Verdünnungsstufen auf den Monolayer der Zellkultur in der MTP pipettiert werden. Zur Trocknungskontrolle werden zwei Virus-Prüfkörper nach der Trocknung für die Dauer des Prozesses im Labor gelagert und anschließend zusammen mit den Prüfkörpern des Hauptversuchs analysiert. Die Elution erfolgt wie oben beschrieben und es werden mit DMEM mit 2 % FBS Verdünnungen bis $10^{-8}$ hergestellt, wobei die einzelnen Verdünnungsstufen auf den Monolayer der Zellkultur in der MTP pipettiert werden.

Zudem werden Validierungskontrollen zur Zytotoxizität, Suszeptibilität und Nachwirkungskontrolle durchgeführt. Zur Kontrolle der Zytotoxizität werden Virusträger mit Belastungssubstanz angeschmutzt und dem Prozess ausgesetzt. Die Prüfkörper werden nach Prozessende eluiert (Rückgewinnungslösung S) und eine Verdünnungsreihe bis $10^{-5}$ angefertigt. Die Zytotoxizität darf den Nachweis der 4 log Reduktion nicht behindern. Zur Kontrolle der Suszeptibilität wird der Monolayer einer MTP mit 100 μl Rückgewinnungslösung S bzw. phosphatgepufferter Salzlösung (PBS) pro well beimpft. Nach einer Stunde Inkubation im Brutschrank bei 37 °C wird das Medium entfernt und jeweils 100 μl der Verdünnungsstufen des Virustiter vor Antrocknung in die wells pipettiert. Zur Nachwirkungskontrolle werden vergleichende Virustitrationen mit Virussuspension, welche 30 min mit Rückgewinnungslösung S bzw. PBS behandelt wurde, durchgeführt.

Tabelle 13 zeigt die Ergebnisse der Positiv- und Validierungskontrollen.

Tabelle 13: Positiv- und Validitätskontrollen viruzide Wirksamkeit

| Kontrolle | $CD_{50}$ | lg-$TCID_{50}$ nach [min] | | |
|---|---|---|---|---|
| | | 0 | 30 | 60 |
| Virus Kontrolle sofort nach Trocknung 1 | n.a. | 6,69 | n.d. | n.d. |
| Virus Kontrolle sofort nach Trocknung 2 | n.a. | 6,88 | n.d. | n.d. |
| Virus Kontrolle nach Einwirkzeit 1 | n.a. | n.d. | n.d. | 6,69 |
| Virus Kontrolle nach Einwirkzeit 2 | n.a. | n.d. | n.d. | 6,75 |
| Suszeptibilität PBS | <1,50 | n.d. | n.d. | 6,88 |
| Suszeptibilität Produkt | <1,50 | n.d. | n.d. | 6,75 |
| Nachwirkungskontrolle PBS | <1,50 | n.d. | 6,13 | n.d. |
| Nachwirkungskontrolle Produkt | <1,50 | n.d. | 6,00 | n.d. |
| Virussuspension 1 | n.a. | 7,25 | n.d. | n.d. |
| Virussuspension 2 | n.a. | 7,50 | n.d. | n.d. |

Dabei bezeichnen $CD_{50}$ die zytotoxische Dosis und TCID50 die infektiöse Dosis der Gewebekultur. Die Abkürzungen n.a. und n.d. stehen für nicht anwendbar bzw. nicht definiert.

Entsprechend der vorstehenden Prüfergebnisse kann mit der angegebenen Ausführungsvariante der Vorrichtung 1 über eine Dauer von 60 min bei 80 °C feuchter Hitze unter geringer Belastung eine ausreichende viruzide Wirkung ($\geq$ 4 log) gegen den Prüfkeim bovines Coronavirus erzielt werden.


**Patentansprüche**

1. Vorrichtung (1) zum Inaktivieren von Mikroorganismen die sich auf und/oder in einem Objekt befinden, insbesondere von Bakterien und/oder Viren, aufweisend eine Grundfläche (2), eine Deckfläche (3) und zumindest zwei Seitenflächen (4), welche die Grundfläche (2) und die Deckfläche (3) miteinander verbinden, wodurch ein Volumen (5) zur Aufnahme des Objektes gebildet ist, **dadurch gekennzeichnet, dass** im Volumen (5) eine Inaktivierungseinrichtung vorgesehen ist, um die Mikroorganismen zu inaktivieren, wobei die Inaktivierungseinrichtung zumindest ein im Volumen (5) vor den Seitenflächen (4) angeordnetes Heizelement (6) zum Beheizen des Volumens (5) und ein Führungsmittel zum Einbringen eines Fluids in das Volumen (5) umfasst, wobei das Heizelement (6) als Elektroheizer ausgebildet ist.

2. Vorrichtung (1) nach Anspruch 1, **dadurch gekennzeichnet, dass** Wasser als Fluid vorgesehen ist.

3. Vorrichtung (1) nach Anspruch 1 oder 2, **dadurch gekennzeichnet, dass** das Heizelement (6) zum Erhitzen des Fluids ausgebildet ist.

4. Vorrichtung (1) nach einem der Ansprüche 1 bis 3, **dadurch gekennzeichnet, dass** das Heizelement (6) als Heizlüfter ausgebildet ist.

5. Vorrichtung (1) nach einem der Ansprüche 1 bis 4, **dadurch gekennzeichnet, dass** eine Basis (7) vorgesehen ist, welche die Grundfläche (2) umfasst und zumindest eine Ausnehmung (8) für ein Hebegerät, insbesondere einen Gabelstapler, aufweist.

6. Vorrichtung nach Anspruch 5, **dadurch gekennzeichnet, dass** die Basis (7) mit zumindest einer Lasche (10) zur Aufnahme zumindest eines ortsfesten Zuganges versehen ist.

7. Vorrichtung (1) nach einem der Ansprüche 1 bis 6, **dadurch gekennzeichnet, dass** eine Grundfläche (2) des Volumens (5) zur Aufnahme einer Flachpalette, bevorzugt einer nach EN 13698-1 genormten Palette, ausgebildet ist.

8. Vorrichtung (1) nach einem der Ansprüche 1 bis 7, **dadurch gekennzeichnet, dass** das Volumen (5) von einer Seite zugänglich und ein Verschluss wie eine Tür (12) vorgesehen ist, mit dem diese Seite verschließbar ist.

9. Vorrichtung (1) nach einem der Ansprüche 1 bis 8, **dadurch gekennzeichnet, dass** ein im Wesentlichen quaderförmiger Rahmen (13) zur Aufnahme der Inaktivierungseinrichtung sowie der Grundfläche (2), Deckfläche (3) und Seitenflächen (4) vorgesehen ist.

10. Vorrichtung (1) nach dem Anspruch 9, **dadurch gekennzeichnet, dass** der Rahmen (13) aus einer Vielzahl von, miteinander stoff- und/oder kraftschlüssig verbundenen, Metallprofilen ausgebildet ist.

11. Vorrichtung (1) nach dem Anspruch 10, **dadurch gekennzeichnet, dass** die Metallprofile hohl ausgebildet sind und einen runden, quadratischen, rechteckigen oder vieleckigen Querschnitt aufweisen.

12. Vorrichtung (1) nach einem der Ansprüche 1 bis 11, **dadurch gekennzeichnet, dass** eine Temperatur in dem Volumen (5) mit der Inaktivierungseinrichtung auf zwischen 60 °C und 100 °C, bevorzugt zwischen 70 °C und 90 °C, besonders bevorzugt 80 °C, einstellbar ist.

13. Verwendung einer Vorrichtung (1) nach einem der Ansprüche 1 bis 12 zum Inaktivieren von Mikroorganismen, insbesondere von Bakterien und/oder Viren, welche sich in und/oder auf einem oder mehreren Objekten befinden.

14. Verfahren zum Inaktivieren von Mikroorganismen, insbesondere von Bakterien und/oder Viren, mit einer Vorrichtung (1) nach einem der Ansprüche 1 bis 12, welche eine Grundfläche (2), eine Deckfläche (3), zumindest zwei Seitenflächen (4) und ein dadurch gebildetes Volumen (5) zur Aufnahme eines Objektes umfasst, wobei auf und/oder in den Objekten befindliche Mikroorganismen bei erhöhten Temperaturen inaktiviert werden, **dadurch gekennzeichnet, dass** das Volumen (5) durch eine im Volumen (5) angeordnete Inaktivierungseinrichtung beheizt und über ein Führungsmittel ein Fluid in das Volumen (5) eingebracht wird.

15. Verfahren nach Anspruch 14, **dadurch gekennzeichnet, dass** das Volumen (5) von zwei Seiten zugänglich und die Oberseite der Grundfläche (2) zur Aufnahme eines Bandförderers (9) ausgebildet ist.

## Claims

1. A device (1) for inactivating microorganisms, in particular bacteria and/or viruses, which are located on and/or in an object having a bottom face (2), a top face (3) and at least two side faces (4) which connect the bottom face (2) and the top face (3) together, whereupon a volume (5) is formed for receiving the object, **characterized in that** an inactivation device is provided in the volume (5) for inactivating the microorganisms, wherein the inactivation device comprises at least one heating element (6) disposed in front of the side faces (4) for heating the volume (5) and a guiding means for introducing a fluid into the volume (5), wherein the heating element (6) is configured as an electric heater.

2. The device (1) as claimed in claim 1. **characterized in that** water is provided as the fluid.

3. The device (1) as claimed in claim 1 or claim 2. **characterized in that** the heating element (6) is configured for heating the fluid.

4. The device (1) as claimed in one of claims 1 to 3. **characterized in that** the heating element (6) is configured as a fan heater.

5. The device (1) as claimed in one of claims 1 to 4. **characterized in that** a base (7) is provided which comprises the bottom face (2) and has at least one recess (8) for a lifting unit, in particular a forklift truck.

6. The device (1) as claimed in claim 5, **characterized in that** the base (7) is provided with at least one bracket (10) for receiving at least one stationary entryway.

7. The device (1) as claimed in one of claims 1 to 6. **characterized in that** the bottom face (2) of the volume (5) is configured to receive a flat palette, preferably a standard EN 13698-1 palette.

8. The device (1) as claimed in one of claims 1 to 7. **characterized in that** the volume (5) is accessible from one side and a closure such as a door (12) is provided with which this side can be closed.

9. The device (1) as claimed in one of claims 1 to 8. **characterized in that** a substantially cuboid frame (13) is provided to receive the inactivation device as well as the bottom face (2), top face (3) and side faces (4).

10. The device (1) as claimed in claim 9. **characterized in that** the frame (13) is constructed from a plurality of metal profiles connected together in a material-bonding and/or force-fitting manner.

11. The device (1) as claimed in claim 10. **characterized in that** the metal profiles are hollow in construction and have a round, square, rectangular or polygonal cross section.

12. The device (1) as claimed in one of claims 1 to 11. **characterized in that** a temperature in the volume (5) can be set with the inactivation device to between 60°C and 100°C, preferably between 70°C and 90°C, particularly preferably 80°C.

13. Use of a device (1) as claimed in one of claims 1 to 12 for inactivating microorganisms, in particular bacteria and/or viruses, which are in and/or on one or more objects.

14. A method for inactivating microorganisms, in particular bacteria and/or viruses, with a device (1) as claimed in one of claims 1 to 12. which comprises a bottom face (2), a top face (3), at least two side faces (4) and a volume (5) formed thereby for receiving an object, wherein microorganisms located on and/or in the objects are inactivated at raised temperatures, **characterized in that** the volume (5) is heated by an inactivation device disposed in the volume (5) and a fluid is introduced into the volume (5) via a guiding means.

15. The method as claimed in claim 14. **characterized in that** the volume (5) is accessible from two sides and the upper side of the bottom face (2) is configured to receive a belt conveyor (9).

**Revendications**

1. Dispositif (1), destiné à inactiver des micro-organismes qui se trouvent sur et / ou dans un objet, notamment des bactéries et / ou des virus, comportant une surface de base (2), une surface de recouvrement (3) et au moins deux surfaces latérales (4), lesquelles assemblent l'une à l'autre la surface de base (2) et la surface de recouvrement (3), suite à quoi est constitué un volume (5) destiné à recevoir l'objet, **caractérisé en ce que** dans le volume (5), il est prévu un système d'inactivation, pour inactiver les micro-organismes, le système d'inactivation comprenant au moins un élément chauffant (6), placé dans le volume (5) à l'avant des surfaces latérales (4), destiné à chauffer le volume (5) et un moyen de guidage, destiné à introduire un fluide dans le volume (5), l'élément chauffant (6) étant conçu sous la forme d'un réchauffeur électrique.

2. Dispositif (1) selon la revendication 1, **caractérisé en ce que** de l'eau est prévue en tant que fluide.

3. Dispositif (1) selon la revendication 1 ou 2, **caractérisé en ce que** l'élément chauffant (6) est conçu pour chauffer le fluide.

4. Dispositif (1) selon l'une quelconque des revendications 1 à 3, **caractérisé en ce que** l'élément chauffant (6) est conçu sous la forme d'un ventilateur chauffant.

5. Dispositif (1) selon l'une quelconque des revendications 1 à 4, **caractérisé en ce qu'**il est prévu un socle (7), lequel comprend la surface de base (2) et au moins un évidement (8) pour un appareil de levage, notamment un chariot élévateur à fourche.

6. Dispositif selon la revendication 5, **caractérisé en ce que** le socle (7) est muni d'au moins une patte (10), destinée à recevoir au moins un accès stationnaire.

7. Dispositif (1) selon l'une quelconque des revendications 1 à 6, **caractérisé en ce qu'**une surface de base (2) du volume (5) est conçue pour recevoir une palette plate, de préférence une palette standardisée selon la norme EN 13698-1.

8. Dispositif (1) selon l'une quelconque des revendications 1 à 7, **caractérisé en ce que** le volume (5) est accessible par un côté et **en ce qu'**il est prévu une fermeture, telle qu'une porte (12), à l'aide de laquelle ce côté peut se fermer.

**9.** Dispositif (1) selon l'une quelconque des revendications 1 à 8, **caractérisé en ce qu'**il est prévu un cadre (13) de forme sensiblement parallélépipédique, destiné à recevoir le système d'inactivation, ainsi que la surface de base (2), la surface de recouvrement (3) et les surfaces latérales (4).

**10.** Dispositif (1) selon la revendication 9, **caractérisé en ce que** le cadre (13) est conçu en une pluralité de profilés métalliques, assemblés les uns aux autres par conjugaison de matières et / ou par complémentarité de force.

**11.** Dispositif (1) selon la revendication 10, **caractérisé en ce que** les profilés métalliques sont conçus de forme creuse et comportent une section transversale ronde, carrée ou rectangulaire n.

**12.** Dispositif (1) selon l'une quelconque des revendications 1 à 11, **caractérisé en ce qu'**une température dans le volume (5) est réglable par le système d'inactivation à entre 60 °C et 100 °C, de préférence, entre 70 °C et 90 °C, de manière particulièrement préférentielle à 80 °C.

**13.** Utilisation d'un dispositif (1) selon l'une quelconque des revendications 1 à 12, pour inactiver des micro-organismes, notamment des bactéries et / ou des virus, qui se trouvent sur et / ou dans un ou plusieurs objets.

**14.** Procédé, destiné à inactiver des micro-organismes, notamment des bactéries et / ou des virus, à l'aide d'un dispositif (1) selon l'une quelconque des revendications 1 à 12, lequel comporte une surface de base (2), une surface de recouvrement (3), au moins deux surfaces latérales (4) et un volume (5) constitué par ces dernières, destiné à recevoir un objet, des micro-organismes se trouvant sur et / ou dans les objets étant inactivés à des températures élevées, **caractérisé en ce que** l'on chauffe le volume (5) à l'aide d'un système d'inactivation placé dans le volume (5) et par l'intermédiaire d'un moyen de guidage, l'on introduit un fluide dans le volume (5).

**15.** Procédé selon la revendication 14, **caractérisé en ce que** le volume (5) est accessible par deux côtés et la face supérieure de la surface de base (2) est conçue pour recevoir un convoyeur à bande (9).

**Fig. 1**

**Fig. 2**

**Fig. 3**

**Fig. 4**

Fig. 5

Fig. 6

**Fig. 7**

**Fig. 8**

**Fig. 9**

**Fig. 10**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- CN 106213836 A

- WO 0038742 A1